# EUROPEAN PATENT APPLICATION

(11) **EP 1 882 692 A1**
(43) Date of publication of application: **30.01.2008**
(21) Application number: 06380219.3
(22) Date of filing: 28.07.2006
(51) Int. Cl.: C07D 491/22, A61K 31/529, A61P 29/00

(54) **Dimethano-[1,3]dioxocino[6,5-D]pyrimidine-spiro derivatives of tetrodotoxin, process for their synthesis and uses thereof in the treatment of pain**

(71) Applicant: Wex Pharmaceuticals Inc., Vancouver, BC V6C 1G8 (CA)
(72) Inventor: Noheda,Marin.P.Instituto Quimica Organica General., E-28006 Madrid (ES); Tabares Cantero.N.Instituto Quimica Organica Gen, E-28006 Madrid (ES)
(74) Representative: ABG Patentes, S.L.

(57) **Abstract**

The present invention relates to a method for the preparation of derivatives of dimethano-[1,3]dioxocino[6,5-d]pyrimidine-spiro derivatives of formula II, III and IV. The present invention also relates to the derivatives of fonnula II, III and IV obtained through the method described, to pharmaceutical compositions comprising the same and to their use as a medicament.

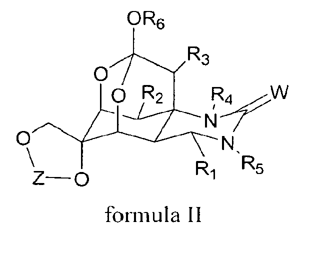

## Description

### FIELD OF THE INVENTION

The present invention relates to a method for the preparation of dimethano-[1,3]dioxocino[6,5-d]pyrimidine-spiro derivatives specially derivatives of Tetrodotoxin. The present invention also relates to the derivatives obtained through the method described, to pharmaceutical compositions comprising the same and to their use as a medicament.

### BACKGROUND OF THE INVENTION

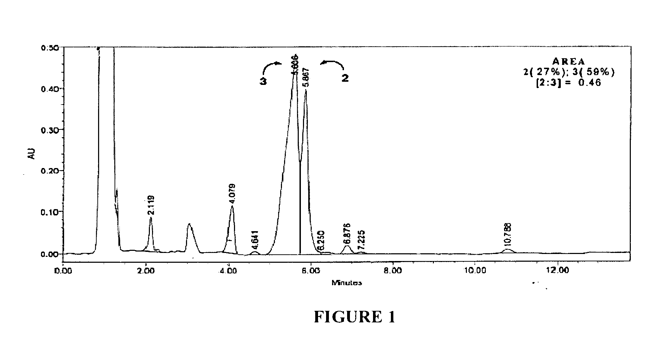

In the present application the following numbering will be used (see figure 1, page 7497 in Sato, K.; Akai, S.; Sugita, N.; Ohsawa, T.; Kogure, T.; Shoji, H.; Yoshimura, J. J. Org. Chem. 2005, 70, 7496-7504):

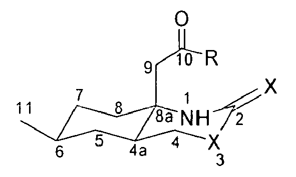

Tetrodotoxin (also abbreviated as "TTX" in the context of this application) or octahydro-12-(hydroxymethyl)-2-imino-5,9:7,10a-dimethano-10aH-[1,3]dioxocino[6,5-d]pyrimidine-4,7,10,11,12-pentol, is a potent Sodium channel blocker, indispensable tool for the study of neurobiology and physiology. Tetrodotoxin was first isolated in 1909 from the ovaries of the puffer fish, and named after the puffer fish family "Tetraodontidae" Despite being a small molecule, it has an extremely complex structure characterised by a dioxaadamantane skeleton, a guanidine residue at C2 which is part of a hemiaminal at C4, and an orthoacid bridge at C10. As a result, TTX has 4 quaternary carbon atoms and 8 sterogenic centers.

It should also be noted that TTX is usually present as a mixture of *inter alia* two possible tautomers: an ortoester and a hydroxylactone (see scheme 1). The ratio of both tautomers depends on the media in which TTX is present.

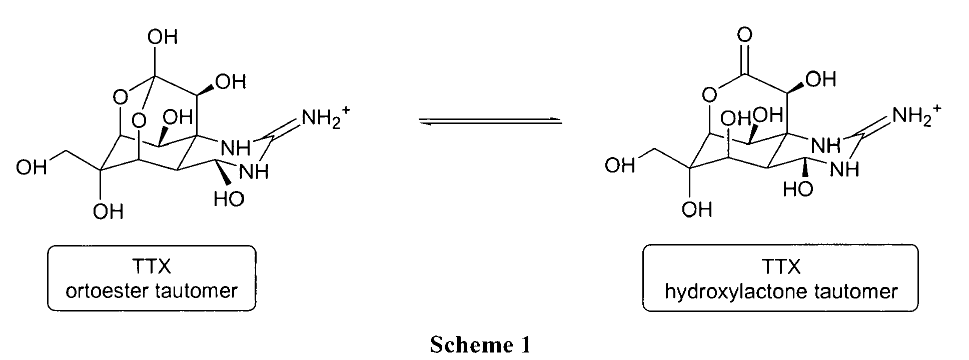

For further discussion regarding tautomeric forms of TTX, reference is made to pages 32-34 of "Tetodotoxin, saxitoxin, and the molecular biology of the sodium channel", Anals of The New York Academy of Science, Vol. 479, Edited by C. Y. Kao and S. R. Levinson; 1986.

Tetrodotoxin blocks diffusion of sodium through the voltage dependent sodium channels, preventing depolarization and propagation of action potentials in nerve cells. The TTX-Na Channel binding site is extremely tight (*K_{d}* = 10⁻¹⁰ nM). Therefore it is used in pharmacological studies related to sodium channel proteins.

Usually, TTX is extracted from marine organisms (e.g. JP 270719 Goto and Takahashi) although a number of synthetic routes have been described (US 6,552,191, US 6,478,966, US 6,562,968 or US 2002/0086997). Tetrodotoxin is a well known compound described for example in WO 02/22129 as systemically acting as analgesic. For one of the many descriptions of TTX it is recommended to turn to e.g. Tu, Anthony (Ed.) Handbook of Natural Toxins, Vol. 3: Marine Toxins and Venoms, 1988, 185-210 as well as to Kao (1966), Pharmacol. Rev. 18:997 - 1049 and others.

In view of the potent activity as a sodium channel blocker there is an ongoing research effort regarding the synthesis of derivatives of TTX. New TTX derivatives could lead to compounds with comparable pharmacological activity, but without the inconveniencies of this potent neurotoxin. US 5,846,975 (included herein by reference) from column 3 line 40 to column 6 line 40, describes a general formula of known TTX derivatives. TTX derivatives are, for example, anhydrotetrodotoxin, tetrodaminotoxin, methoxytetrodotoxin, ethoxytetrodotoxin, deoxytetrodotoxin and tetrodonic acid, 6 epitetrodotoxin, 11-deoxytetrodotoxin as well as the hemilactal type TTX derivatives (e.g. 4-*epi-TTX, 6-epi-TTX,* 11*-deoxy-*TTX*,* 4-*epi*-11-*deoxy*-TTX, TTX-8-*O*-hemisuccinate, chiriquitoxin, 11-*nor*-TTX-6(S)-ol, 11-*nor*-TTX-6(R)-ol, 11-*nor*-TTX-6,6-diol, 11-*oxo-*TTX and TTX-11-carboxylic acid), the lactone type TTX derivatives (e.g. 6*-epi-*TTX (lactone), 11-*deoxy*-TTX (lactone), 11-*nor*-TTX-6(S)-ol (lactone), 11-*nor*-TTX-6(R)-ol (lactone), 11-*nor*-TTX-6,6-diol (lactone), 5-*deoxy*-TTX, 5,11*-dideoxy-*TTX*,* 4*-epi-*5,11-*didroxy-*TTX*,* 1*-hydroxy-*5,11*-dideoxy-*TTX*,* 5,6,11-*trideoxy*-TTX and *4-epi-5,6,11-trideoxy-*TTX) and the 4,9-anhydro type TTX analogs (e.g. 4,9*-anhydro-*TTX, 4,9-*anhydro-*6*-epi-*TTX*,* 4,9*-anhydro-*11*-deoxy-*TTX*,* 4,9-*anhydro*-TTX-8-*O*-hemisuccinate, 4,9*-anhydro-*TTX*-*11-*O*-hemisuccinate). The typical derivatives of TTX possess only 1/8 to 1/40 of the toxicity of TTX in mice, based upon bioassay in mice. It has been observed that these derivatives produce joint action, and do not interact adversely.

Woodward et. al. (Pure Appl. Chem. 1964, 9, 49-74), from now on "Woodward's paper", describes two 6,11-isopropylidine derivatives of TTX: 6,11-O-isopropylidene-4,9-anhydrotetrodotoxin-hydrochloride and 4-O-Methyl-6,11-O-isopropylidenetetrodotoxin-hydrochloride (Rajappa hydrochloride and Gougoutas hydrochloride, respectively):

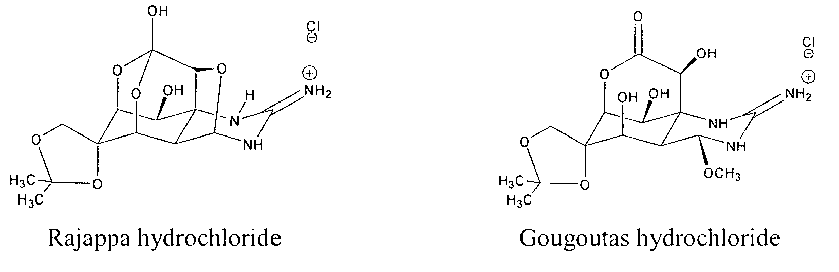

Although Woodward's paper does not specifically discloses the method for obtaining both compounds, a general description of the method used is disclosed in page 37 of "Tetodotoxin, saxitoxin, and the molecular biology of the sodium channel", Anals of The New York Academy of Science, Vol. 479, Edited by C. Y. Kao and S. R. Levinson; 1986. According to the latest, Gougoutas hydrochloride was obtained by first bubbling HCl to a solution of anhydrous methanol/acetone and then storing TTX in the resulting anhydrous solution of methanol/acetone/HCl at room temperature. Rajappa hydrochloride was obtained by first bubbling HCl to a solution of anhydrous acetone and then storing TTX in the resulting anhydrous solution of acetone/HCl at room temperature, in the absence of methanol. Cristalographic data is provided for Gougoutas hydrochloride.

The above method for obtaining Gougoutas hydrochloride is described in more detail in Nachman, R.J. Ph D thesis, 1981, Stanford University, Standford, California (from now on, "Nachman's thesis"). The process comprises adding 10.15 mg (31.8 µmoles) of tetrodotoxin to a solution of 1.75 ml of anhydrous 0.3 N methanolic hydrogen chloride and 1.00 ml of distilled acetone. The TTX quickly dissolved and the solution was stored 48 hours. The solution was then stored for an additional 72 hours over methanol saturated with potassium hydroxide. The volume of solution had been reduced by evaporation to 0.5 ml and was filled with crystals. The crystal suspension was centrifuged and the supernatant was drawn off with a 50µl syringe. The crystals were both washed with 0.15 ml of cold methanol and centrifuged a total of three times to obtain 7.67 mg of crystalline gogoutas hydrochloride (65% yield).

### SUMMARY OF THE INVENTION

As mentioned above, there is an ongoing research effort for providing TTX derivatives and prodrugs and alternative synthetic approaches to said derivatives of TTX.

Thus, according to a first aspect, the present invention is directed to a process for the synthesis of a compound of formula II as defined below comprising the step of reacting a compound of formula I as defined below, with a carbonyl or boron reagent as defined below, in the presence of a substantially anhydrous acid solution which has been generated in situ .

According to a further aspect, the present invention is directed to a process for the synthesis of a compound of formula III as defined below comprising the step of reacting a compound of formula I with said carbonyl or boron reagent, in the presence of a substantially anhydrous acid solution which has been generated in situ.

According to a further aspect, the present invention is directed to a process for the synthesis of a compound of formula IV as defined below comprising the step of reacting a compound of formula I with said carbonyl or boron reagent, in the presence of a substantially anhydrous acid solution which has been generated in situ.

According to a further aspect, the present invention is directed to said compound of formula II or its tautomers or enantiomers or solvates or salts or mixtures thereof.

According to a further aspect, the present invention is directed to said compound of formula III or its tautomers or enantiomers or solvates or salts or mixtures thereof.

According to a further aspect, the present invention is directed to said compound of formula IV or its tautomers or enantiomers or solvates or salts or mixtures thereof.

According to a further aspect, the present invention is directed to a pharmaceutical composition comprising said compound of formula II, or a pharmaceutically acceptable salt or solvate or tautomer or enantiomer thereof, and at least one pharmaceutically acceptable carrier.

According to a further aspect, the present invention is directed to a pharmaceutical composition comprising said compound of formula III, or a pharmaceutically acceptable salt or solvate or tautomer or enantiomer thereof, and at least one pharmaceutically acceptable carrier.

According to a further aspect, the present invention is directed to a pharmaceutical composition comprising said compound of formula IV, or a pharmaceutically acceptable salt or solvate or tautomer or enantiomer thereof, and at least one pharmaceutically acceptable carrier.

According to a further aspect, the present invention is directed to the use of said compound of formula II for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

According to a further aspect, the present invention is directed to the use of said compound of formula III for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

According to a further aspect, the present invention is directed to the use of said compound of formula IV for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

### BRIEF DESCRIPTION OF THE FIGURES

FIGURE I is an HPLC chromatogram of an aliquot (time 8 days) of the preparation reaction of 6,11-*O*-isopropylidene-4,9-anhydrotetrodotoxin hydrochloride **(2)** and 6,11-*O*-isopropylidene-4,9-anhydrotetrodotoxin-hydrochloride **(3).** The arrows show the peaks assigned to compounds **2** and **3** (ratio [**2:3**] = 0.46) by means of their corresponding mass spectra. See Example 1.
FIGURE 2 is a ¹H-RMN (Methanol-d₄) spectrum of a mixture of compounds **2** and **3** (ratio [**2**:**3**], aprox. 0.5; ratio calculated by the proportion of the integrals corresponding to H(4) for compounds **2** and **3**, respectively). Keys: #, signal assigned to H(4) of compound **2**; *, signal assigned to H(4) of compound **3**. See Example 1.
FIGURE 3 is a mass spectrum of a mixture of compounds **2** and **3** (ratio [**2:3**], aprox. 0.5; determinated from ¹H-RMN and HPLC). The arrows show the peaks assigned to compounds **2** and **3.** See Example 1.
FIGURE 4 is a HPLC description of an aliquot of the formation reaction of the mixture of **2** and **3** at a time of 26 hours (t1) (The arrows show the peaks assigned to compounds **2** and **3** supported by their corresponding mass spectra). See Example 1.
FIGURE 5 is a HPLC description of an aliquot of the formation reaction of the mixture of **2** and **3** at a time of 144 hours (t2) (The arrows show the peaks assigned to compounds **2** and **3** supported by their corresponding mass spectra). See Example 1.
FIGURE 6 is a HPLC description of an aliquot of the formation reaction of the mixture of **2** and **3** at a time of 192 hours (t3) (The arrows show the peaks assigned to compounds **2** and **3** supported by their corresponding mass spectra). See Example 1.
FIGURE 7 is a ¹H-RMN (Methanol-d₄) spectrum of 4-*O*-Methyl-6,11-*O-*isopropylidenetetrodotoxin-hydrochloride **(4)**. See Example 2.
FIGURE 8 is a Mass spectrum of compound **4**. See Example 2.
FIGURE 9 is an HPLC description of an aliquot of the formation reaction of the compound **4** at a time of 7 hours (t1) (the arrows show the peaks assigned to compounds TTX **(1), 2, 3** and **4** supported by their corresponding mass spectra). See Example 2.
FIGURE 10 is an HPLC description of an aliquot of the formation reaction of the compound **4** at a time of 120 hours (t2) (the arrows show the peaks assigned to compounds **2, 3** and **4** supported by their corresponding mass spectra). See Example 2.
FIGURE 11 is an HPLC description of an aliquot of the formation reaction of the compound **4** at a time of 168 hours (t3) (the arrows show the peaks assigned to compounds **2**, **3** and **4** supported by their corresponding mass spectra). See Example 2.
FIGURE 12 is an HPLC description of an aliquot of the formation reaction of the compound **4** at a time of 216 hours (t4) (the arrows show the peaks assigned to compounds **2, 3** and **4** supported by their corresponding mass spectra). See Example 2.
FIGURE 13 is an HPLC description of an aliquot of the formation reaction of the compound **4** at a time of 888 hours (t5) (the arrows show the peaks assigned to compounds **2, 3** and **4** supported by their corresponding mass spectra). See Example 2.

### DETAILED DESCRIPTION OF THE INVENTION

### Definitions

It will be appreciated that for the purposes set out herein, the compounds of formula I, II, III or IV can be optionally in the form of their racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a pharmaceutically acceptable salt, or in form of a solvate, especially a hydrate. The process of the invention does not usually involve a change in the spatial configuration of any of the stereocenters of the molecule. Thus, if the starting material is a racemate, the product of the invention will usually be a racemate of a compound of formula II, III or IV. However, it should be noted that partial or total epimerization of one or more stereogenic centers is also possible.

In order to facilitate the comprehension of the present invention, the meanings of some terms and expressions as used in the context of the invention are included herein.

**"Heterocyclyl"** refers to a stable 3- to 15- membered ring which consists of carbon atoms and from one to five heteroatoms selected from the group consisting of nitrogen, oxygen, and sulphur, preferably a 5- to 10-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quatemized; and the heterocyclyl radical may be partially or fully saturated or aromatic. Examples of such heterocycles include, but are not limited to, azepines, benzimidazole, benzothiazole, furan, isothiazole, pyridine, imidazole, indole, piperidine, pyrrolidine, piperazine, purine, quinoline, isoquinoline, quinoxaline, thiadiazole, tetrahydrofuran, phtalamide.

**"Aryl"** refers to an aromatic hydrocarbon radical with 6 to 20, preferably 6 to 10, carbon atoms, such as phenyl, naphthyl or anthracyl.

**"Aralkyl"** refers to an aryl group linked to an alkyl group such as benzyl and phenethyl.

**"Alkyl"** refers to a straight or branched hydrocarbon chain radical consisting of carbon and hydrogen atoms, containing no saturation, having 1-12, preferably one to six carbon atoms, and which is attached to the rest of the molecule by a single bond, e. g., methyl, ethyl, n-propyl, i-propyl, n-butyl, t-butyl, n-pentyl, etc.

**"Alkoxyl"** refers to a radical of the formula -O-alkyl, -O-akenyl, -O-aryl or -O-aralkyl, wherein alkyl, alkenyl, aryl and aralkyl are as previously defined, e. g., methoxy, ethoxy, propoxy, phenoxy, vinyl ethyl ether, benzyloxy, etc.

**"Alkenyl"** refers to a straight or branched hydrocarbon chain radical consisting of carbon and hydrogen atoms, containing at least one unsaturation, having 1-12, preferably one to six carbon atoms, and which is attached to the rest of the molecule by a single bond.

"Cycloalkenyl" refers to a carbocyclic ring having from 3 to 8 carbon atoms and at least one unsaturation.

**"Cycloalkyl"** refers to a saturated carbocyclic ring having from 3 to 8 carbon atoms.

All of the above groups (heterocyclyl, aryl, aralkyl, alkyl, alkoxyl, alkenyl, cycloalkenyl and cycloalkyl) may be substituted or unsubstituted. References herein to **substituted groups** in the compounds of the present invention refer to the specified moiety that may be substituted at one or more available positions by one or more suitable groups, e. g., halogen such as fluoro, chloro, bromo and iodo ; alkyl; aryl; protecting groups; cyano; hydroxyl; nitro; azido ; alkanoyl such as a C₁₋₆ alkanoyl group such as acyl and the like; carboxiamides of formula -CONH₂, -CONHR', -CONR'R", wherein R' and R" are independently selected from substituted or unsubstituted alkyl, substituted or unsubstituted cycloalkyl, substituted or unsubstituted alkenyl, substituted or unsubstituted cycloalkenyl, substituted or unsubstituted aryl, substituted or unsubstituted aralkyl or substituted or unsubstituted heterocyclyl; or R' and R" together form a substituted or unsubstituted heterocycle, substituted or unsubstituted cycloalkyl or substituted or unsubstituted cycloalkenyl; carboxylic acid (-COOH); carboxylate of formula -C(=O)-O-alkyl, wherein alkyl is as defined above; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

**"Halogen"** refers to fluoro (-F), chloro (-C 1), bromo (-Br) or iodo (-I).

**"Protecting group"** refers to a group that blocks an organic functional group and can be removed under controlled conditions. Protecting groups, their relative reactivity and conditions under which they remain inert are known to the skilled person. Reference is made to Greene and Wuts "Protective Groups in Organic Synthesis", John Wiley & Sons, Inc., New York, 1999.

For example, **"amino protecting group"** refers to a group that blocks the -NH₂ function for further reactions and can be removed under controlled conditions. The amino protecting groups are well known in the art, representative protecting groups are carbamates, e.g. carbamates of formula -C(=O)OR"', amides, e.g. amides of formula -C(=O)R"', such as substituted or unsubstituted acetates or silyl moieties of formula -Si(R"')₃, wherein R"' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl. Also different alkyl moeties may serve as amino protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amino protecting groups can be found in reference books such as Greene and Wuts' "Protective Groups in Organic Synthesis", John Wiley & Sons, Inc., New York, 1999.

Further, **"hydroxyl protecting group"** refers to a group that blocks the -OH function for further reactions and can be removed under controlled conditions. The hydroxyl protecting groups are well known in the art, representative protecting groups are:
- silyl ethers of formula -Si(R"')₃, such as trimethylsilyl ether (also represented as "TMS"), triethylsilyl ether, tert-butyldimethylsilyl ether (also represented as "TBDMSO"), tert-butyldiphenylsilyl ether, tri-isopropylsilyl ether, diethylisopropylsilyl ether, thexyldimethylsilyl ether, triphenylsilyl ether, di-tert-butylmethylsilyl ether, and other bifunctional silyl protecting groups such as 1,1,3,3-tetraisopropyldisiloxane-1,3-diyl;
- alkyl ethers of formula -R"', such as methyl ether, tert-butyl ether, benzyl ether, p-methoxybenzyl ether , 3,4-dimethoxybenzyl ether, trityl ether; allyl ether;
- alkoxymethyl ethers of formula -CH₂-O-R"', such as methoxymethyl ether, 2-methoxyethoxymethyl ether, benzyloxymethyl ether, p-methoxybenzyloxymethyl ether, 2-(trimethylsilyl)ethoxymethyl ether. The oxygen atom may be replaced by a sulphur atom to form an alkylthiomethyl ether of formula -CH₂-S-R', such as methylthiomethyl ether. Tetrahydropyranyl and related ethers are also commonly used as hydroxyl protecting groups;
- esters of formula -C(=O)R'", such as acetate ester, benzoate ester; pivalate ester; methoxyacetate ester; chloroacetate ester; levulinate ester;
- carbonates of formula -C(=O)-O-R"', such as benzyl carbonate, p-nitrobenzyl carbonate, tert-butyl carbonate, 2,2,2-trichloroethyl carbonate, 2-(trimethylsilyl)ethyl carbonate, allyl carbonate; or
- sulphates such as SO₃py.

In all the above formula R' is as defined above.

Additional examples of hydroxyl protecting groups can be found in reference books such as Greene and Wuts' "Protective Groups in Organic Synthesis", John Wiley & Sons, Inc., New York, 1999.

The term **"pharmaceutically acceptable"** refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopoeia or other generally recognized pharmacopoeia for use in animals, and more particularly in humans.

The term **"salt"** refers to the compounds of the present invention having a ionic form or a charge and are coupled to a counter ion (cation or anion), or are in solution. Complexes of the active compounds of formula II, III or IV are also included, particularly ionic complexes between the compounds of formula II, III or IV and other molecules. The term salt as used herein also encompasses Zwitterion forms, that is, forms having both, acidic and basic groups in the same molecule. Thus, when suitable, they can be represented as neutral molecules or diionic forms. For example, TTX can be represented as the neutral form of diionic form: 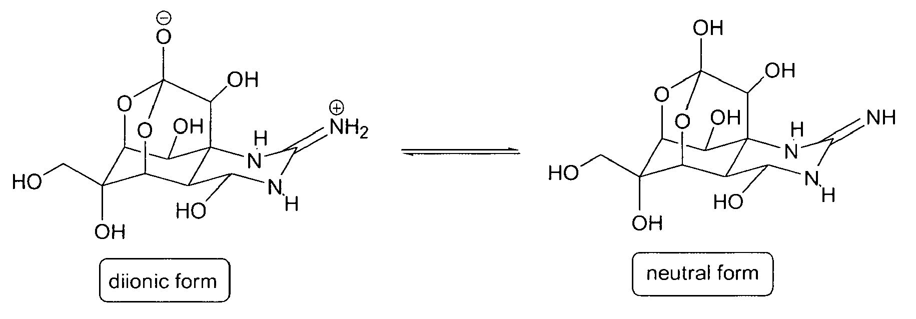 Also, the compounds of formula II and IV of the invention may be present as Zwitterion or as neutral forms.

The term **"pharmaceutically acceptable salts"** refers to any pharmaceutically acceptable salt, which, upon administration to the recipient is capable of providing (directly or indirectly) the compounds as described herein.

For instance, pharmaceutically acceptable salts of compounds provided herein may be acid addition salts, base addition salts or metallic salts, and they can be synthesized from the parent compound which contains a basic or an acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term **"carrier"** refers to a diluent, adjuvant, excipient, and/or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous saline solution, or dextrose or glycerol solutions are preferably employed as carriers, particularly for injectable solutions. Suitable pharmaceutical carriers are described in "Remington's Pharmaceutical Sciences" by E.W. Martin.

The term **"solvate"** according to this invention is to be understood as meaning any form of the active compound according to the invention which has another molecule (most likely a polar solvent) attached to it via non-covalent bonding. Examples of solvates include hydrates and alcoholates, e.g. methanolate.

### Process for the synthesis of compounds of formula II

In the continuous research effort of the inventors with regard to TTX, its derivatives or prodrugs thereof, the synthesis of a compound of formula II was attempted. Different experiments where performed with different acids. Surprisingly, in situ generation of the acid under anhydrous conditions was effective for obtaining the desired compound of formula II.

Thus, as mentioned above, the first aspect of the present invention is a process (hereinafter referred to as process of the invention) for the synthesis of a compound of formula II: 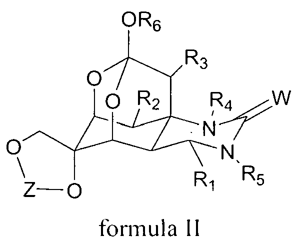 wherein
R₁ is selected from the group consisting of -H, -OH, -OC(=O)R^{a}, -OR^{a}, alkyl,-NH₂, -NHR^{b} and -NR^{b}R^{c}, wherein R^{a}, R^{b} and R^{c} are independently selected from an alkyl group;
R₂ and R₃ are independently selected from the group consisting of -H, -OH and-OC(=O)R^{a}, -OR^{b}, wherein R^{a} and R^{b} are as previously defined;
R₄ and R₅ are independently selected from the group consisting of -H and amino protecting group;
R₆ is selected from the group consisting of -H, a negative charge and a hydroxyl protecting group;
W is selected from the group consisting ofNH, ⊕NH₂, O and S; and
Z is selected from the group consisting of
   (R₇)(R₈)C< wherein R₇ and R₈ are each independently selected from the group consisting of alkyl, cycloalkyl, alkenyl, cycloalkenyl, alcoxyl, heterocyclyl, aryl and aralkyl; or R₇ and R₈ together form a group selected from the group consisting of cycloalkyl, heterocyclyl and cycloalkenyl; and -(R₉)B<, wherein R₉ is selected from the group consisting of alkyl, cycloalkyl, alkenyl, cycloalkenyl, heterocyclyl, aryl and aralkyl;
or its salts, in particular pharmaceutically acceptable salts, or tautomers, solvates or enantiomers thereof;
comprising the step of reacting a compound of formula I 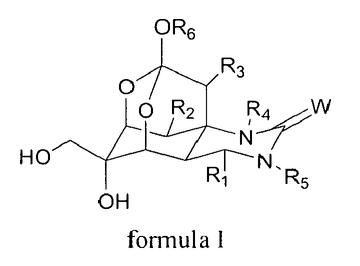 wherein
R₁, R₂, R₃, R₄, R₅, R₆ and W are as defined in formula II;
with a carbonyl or boron reagent selected from the group consisting of
- a carbonyl containing molecule of formula (R₇)(R₈)C=O or its hydrates or alkyl or aryl acetals or hemiacetals, wherein R₇ and R₈ are as previously defined;
- a boron derivative of formula R₉B(OR₁₀)₂, wherein R₁₀ are both independently selected from the group consisting of -H, alkyl, cycloalkyl, alkenyl, cycloalkenyl, heterocyclyl, aryl and aralkyl;
- a boron derivative of formula R₉B=O; and
- a boron derivative of formula 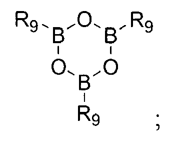 wherein R₉ is as defined above;
in the presence of a substantially anhydrous acid solution which has been generated in situ.

The process of the invention yields the compounds of formula II in good yield. For purposes of the present invention, "generating in situ a substantially anhydrous acid solution" or "a substantially anhydrous acid solution which has been generated in situ" makes reference to the methods known by the skilled person to generate acids in the reaction media from appropriate precursors of the acid.

In one embodiment said methods involve the addition of an alcohol and of an acid precursor which react to yield the corresponding acid.

According to a preferred embodiment, said alcohol is an alcohol of formula R₁₁OH, wherein R₁₁ is selected from the group consisting of alkyl, cycloalkyl, alkenyl, cycloalkenyl, heterocyclyl, aryl and aralkyl.

According to an embodiment, said acid precursor is selected from the group consisting of
- an acyl halide of formula R₁₂COX, wherein
   X is selected from the group consisting of F, Cl, Br or I; and
   R₁₂ is selected from the group consisting of alkyl, cycloalkyl, alkenyl, cycloalkenyl, heterocyclyl, aryl and aralkyl;
- an acid anhydride of formula R₁₂C(=O)O(O=)CR₁₂, wherein each of R₁₂ can be the same or different as defined above; and
- an acid anhydride of formula R₁₃(O=)₂SOS(=O)₂R₁₃, wherein each of R₁₃ are independently selected from the group consisting of alkyl, preferably halogen substituted alkyl, aryl and aralkyl.

For economy reasons, R₁₁ is usually an alkyl group, preferably a C₁-C₆ alkyl, such as methyl or ethyl. However, the skilled person is aware that any alcohol capable of reacting with a given acyl halide or acid anhydride is suitable for the purposes of the present invention, i. e. generating in situ a substantially anhydrous acid solution.

Also, for economy reasons R₁₂ or R₁₃ are usually an alkyl group, preferably a C₁-C₆ alkyl, such as methyl or ethyl. Also, the skilled person is aware that any acid precursor capable of reacting with a given alcohol is suitable for the purposes of the present invention. According to an alternative embodiment, R₁₃ is -CF₃ or p-tolyl, so that the anhydride of formula R₁₃(O=)₂SOS(=O)₂R₁₃ is trifluoromethanesulfonic anhydride or p-tolylsulfonic anhydride respectively.

The pH usually achieved by the process of the invention is preferably below 4.5, more preferably bellow 4 and more preferably below 3.

For the purposes of the present invention "substantially anhydrous" refers to solutions or reaction media substantially free of water. The skilled person is aware of the precautions needed in each case to obtain a substantially anhydrous reaction media. It is also immediately apparent to the skilled person, that it is almost impossible to generate a reaction media which is completely free of water. The amount in which water is present depends to a large extend of the material available in the moment of performing the reaction. Further, the presence of small amounts of water does not completely hamper the reaction, but lowers the yields of the desired compounds of formula II, III or IV obtained.

It is also evident to the skilled person that removing water generated during the reaction is advantageous. Means for removing water from reaction media are known in the art, e.g. Dean-Stark apparatus or molecular sieves.

As a result of the process of the invention, the hydroxyl groups on C6 and C11 are protected (group "Z" in the compounds of formula II). There are a number of protecting groups for 1,2-diols described in the art. Cyclic acetals are widely used. The most common reagent used is the appropriate carbonyl containing molecule. However, it will be immediately apparent to the skilled person the possibility of using equivalents or precursors (synthetic equivalents) of a carbonyl containing molecule in order to effect the reaction. Synthetic equivalents are functional groups used in organic synthesis which have a similar structure and perform substantially the same function. The present patent application encompasses all such synthetic equivalents or equivalents of the carbonyl group. For example, it would be obvious to the skilled person the use of dialkylacetals as synthetic equivalents of ketones. The skilled person can easily find in the literature which groups are equivalents of the carbonyl group for the purposes of process of the invention; for example, see Greene and Wuts' "Protective Groups in Organic Synthesis", John Wiley & Sons, Inc., New York, 1999. Some examples of synthetic equivalents of a carbonyl group are compounds having the following formulas: 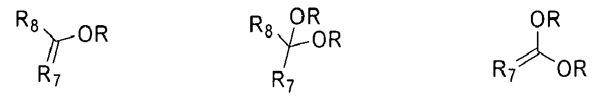 wherein R₇ and R₈ have the same meaning as above, and R is usually an alkyl group, although it may also be selected from the group consisting of aryl, aralkyl, heterocyclyl, alkenyl, cycloalkyl or cycloalkenyl, or both R groups together form a cycloalkyl or cycloalkenyl group. The skilled person is aware these are the most common equivalents of a carbonyl containing molecule, although other equivalents are well known in the art, such as hydrates, or hemiacetals or orthoacetals or imines or enamines. Thioacetals are also commonly used as carbonyl equivalents.

According to a preferred embodiment, (R₇)(R₈)C=O is selected from the group consisting of formaldehyde, acetaldehyde, 2,2-dimethylpropylaldehyde, 3,3-dimethylbutirylaldehyde, 3,3-dimethyl-2-butanone, 4'-methoxyacetophenone, acrolein, acetone, ethyl acetate, methyl acetate, ethyl formate, methyl formate and 3H-Isobenzofuran-1-one, or the hydrates or alkyl or aryl acetals or hemiacetals thereof. According to a preferred embodiment, (R₇)(R₈)C=O is acetone.

The boron reagents of the invention are commercially available or readily prepared in different forms. Thus, depending on the nature of the specific boron reagent it may be in the form of a compound of formula R₉B(OR₁₀)₂, a compound of formula R₉B=O or a compound of formula 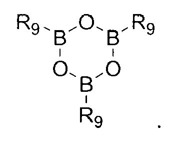 wherein R₉ is as previously defined.

According to further embodiment, the compound of formula R₉B=O is selected from the group consisting of butylboronic acid and phenylboronic acid.

The compounds of formula I used as starting materials for the reaction may be synthesized by processes known in the art. As mentioned above US 6,552,191, US 6,478,966, US 6,562,968 or US 2002/0086997 describe different synthesis of TTX. Also, European patent application EP05077580.8, in the name of the applicant, discloses the synthesis of a compound of formula I. All the previous references are herein incorporated by reference.

It is known that the compounds of formula I (TTX and its derivatives) in solution, usually represented as an orthoester, are in equilibrium with the corresponding hydroxylactone (See scheme 2). The relative proportion of each one depends on the nature of each derivative and on the physico-chemical conditions of the solution (temperature, polarity of the solvent, pH, etc). Therefore, it can be considered that representing TTX as an orthoester or as a hydroxylactone is equivalent.

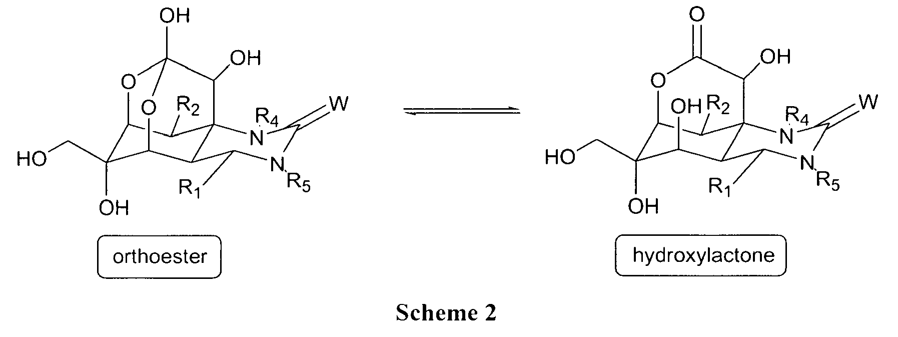

In the same way as above, the compounds of formula II wherein R₆ is hydrogen or a negative charge, may be present as an equilibrium of two tautomeric forms as shown in scheme 3: 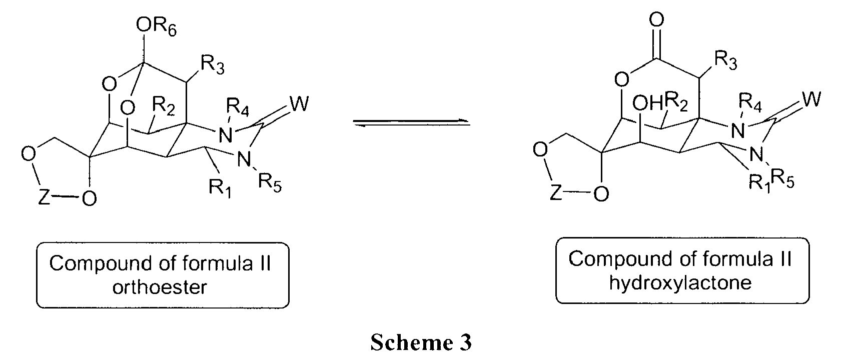 wherein R₁, R₂, R₃, R₄, R₅, Z and W are as defined above for the compounds of formula II. Thus, for the purposes of the present invention the representation of a compound of formula II as an orthoester is equivalent to the representation as a hydroxylactone.

### Process for the synthesis of compounds of formula III and IV

As mentioned above, in the process of the invention it is possible to obtain the compounds of formula II. However, following the same principle it is also possible to carry out the reaction so as to yield the compounds of formula III and IV described hereinafter.

Thus, according to a further aspect, the present invention is directed to a process for the synthesis of a compound of formula III 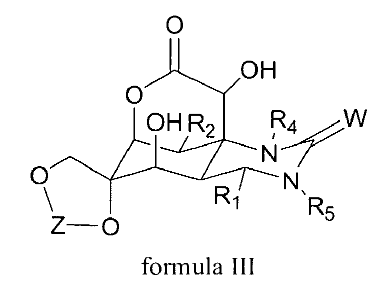 wherein
R₂, R₄, R₅ and Z are as defined in claim 1;
R₁ is alcoxyl; and
W is selected from the group consisting of -NH, O and S;
or its salts, in particular pharmaceutically acceptable salts, or tautomers, solvates or enantiomers thereof;
comprising the step of reacting a compound of formula I, wherein R₃ is -OH, with a carbonyl or boron reagent of formula (R₇)(R₈)C=O or of formula R₉B(OR₁₀)₂, or of formula R₉B=O or of formula 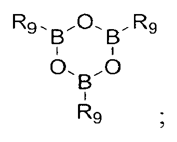 wherein the compound of formula I and the carbonyl or boron reagent are as defined in claim 1 in the presence of a substantially anhydrous acid solution which has been generated in situ and an alcohol, said alcohol being in excess with regard to the carbonyl or boron reagent.

In the same way as explained above, the compounds of formula III may be present as an equilibrium of two tautomeric forms as shown in scheme 4: 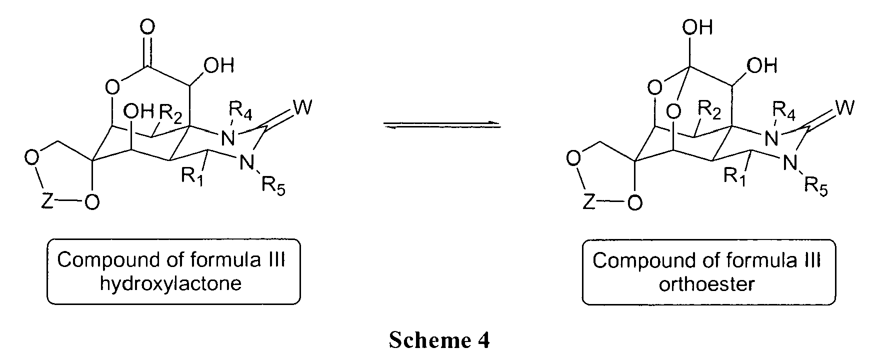 wherein R₁, R₂, R₄, R₅, Z and W are as defined above for the compounds of formula III. Thus, for the purposes of the present invention the representation of a compound of formula III as an orthoester is equivalent to the representation as a hydroxylactone.

According to a further aspect, the present invention is directed to a process for the synthesis of a compound of formula IV 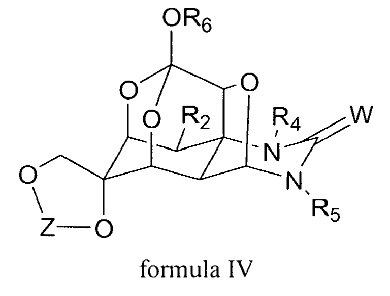 wherein
R₂, R₄, R₅, R₆ and Z are as defined in claim 1; and
W is selected from the group consisting of -NH, O and S;
or its salts, in particular pharmaceutically acceptable salts, or tautomers, solvates or enantiomers thereof;
comprising the step of reacting a compound of formula I, wherein R₃ is -OH, with a carbonyl or boron reagent of formula (R₇)(R₈)C=O or of formula R₉B(OR₁₀)₂ or of formula R₉B=O or of formula 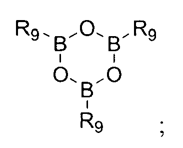 wherein the compound of formula I and the carbonyl or boron reagent are as defined in claim 1, in the presence of a substantially anhydrous acid solution which has been generated in situ and an alcohol, said alcohol being in equimolar ratio with regard to the carbonyl or boron reagent.

In the same way as above, the compounds of formula IV wherein R₆ is hydrogen or a negative charge, may be present as an equilibrium of two tautomeric forms as shown in scheme 5: 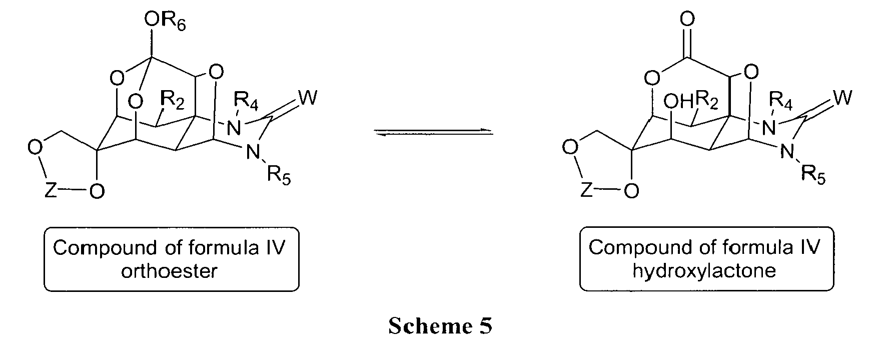 wherein R₂, R₄, R₅, Z and W are as defined above for the compounds of formula IV. Thus, for the purposes of the present invention the representation of a compound of formula IV as an orthoester is equivalent to the representation as a hydroxylactone.

Of course, the skilled person is aware that the term "equimolar" ratio stands for the situation in which two or more compounds are in the same molar amount. However, one of the compounds may be added in slightly different amounts for different reasons. For example, one of the compounds could be more stable than the other, and, in order to achieve the same effective amount of both in the reaction media, it is possible to add less amounts of the more stable compound. Differences may also arise due to the intrinsic limits of the measuring devices commonly used in a laboratory. Therefore, for the purposes of the invention, the term "equimolar" also encompasses small variations in the molar proportions of the components.

In the process for the synthesis of the compound of formula III and in the process for the synthesis of a compound of formula IV, the alcohol and the precursor of the acid have a function which is similar to that performed in the case of the synthesis of the compounds of formula II. Therefore, according to an embodiment of the invention, said alcohol is an alcohol of formula R₁₁OH, wherein R₁₁ is selected from alkyl, cycloalkyl, alkenyl, cycloalkenyl, heterocyclyl, aryl and aralkyl, preferably C₁-C₆ alkyl.

According to a further embodiment, the compound of formula III or IV obtained is in the form of a salt, preferably a salt of and acid selected from the group consisting of XH, R₁₂C(=O)OH and R₁₃(O=)₂SOH, wherein X, R₁₂ and R₁₃ are as defined above. Thus, said salts may be a result of the acid generated during the process of the invention. However, different salts may be generated through a salt exchange reaction by dissolving the compound in a suitable solvent in the presence of an excess of desired ion.

Thus, following a similar process it is possible to obtain compounds of formula II, III or IV from said compound of formula I. There are essentially three possible versions of the reaction.
a) Generation in situ of the substantially anhydrous acid solution can be performed regardless of the nature of the acid precursor. As explained below and in the examples, the reaction time may be controlled in order to either obtain the compounds of II or the compounds of formula III or IV.
b) On the other hand, when an alcohol and a suitable acid precursor are used for generating in situ the substantially anhydrous acid solution, but the alcohol is in equimolar ratio with regard to the acid precursor, the resulting compound is a compound of formula II or a compound of formula IV. According to an embodiment of the invention, short reaction times yield the compound of formula II as the major product of the reaction. The compound of formula II may act as an intermediate towards the compounds of formula III and IV. Therefore, if the reaction is left sufficient time, the major product will be the compound of formula IV. See scheme 6. For further discussion, see Example 1.
c) When an alcohol and a suitable acid precursor are used for generating in situ the substantially anhydrous acid solution and the alcohol is in excess with regard to the acid precursor, the resulting compound is a compound of formula II or a compound of formula III. In a similar way, according to an embodiment of the invention short reaction times yield the compound of formula II as the major product of the reaction. If the reaction is left sufficient time, the major product will be the compound of formula III. See scheme 7. For further discussion, see Example 2.

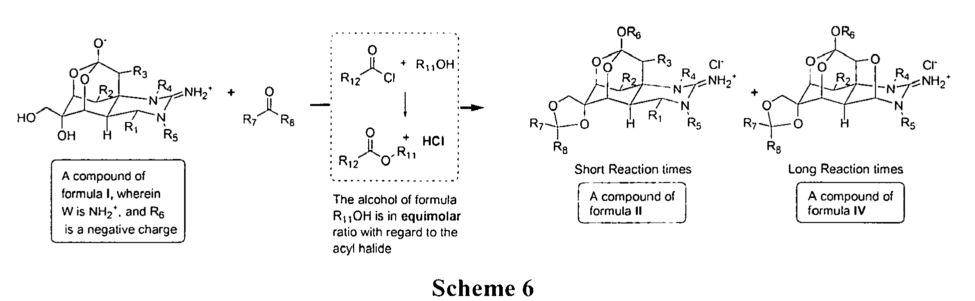

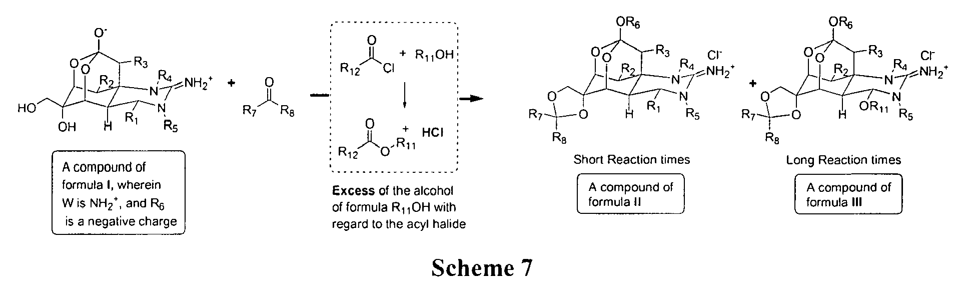

According to an embodiment of the invention, the reaction time of the process of the invention is less than 100 hours, preferably less than 50 hours, more preferably less than 40 hours, more preferably less than 30 hours, more preferably less than 25 hours.

On the other hand, according to an embodiment of the invention, the time needed to complete the reaction and obtain either the compound of formula III or the compound of formula IV as major product is more than 5 hours, preferably more than 10 hours, more preferably more than 20 hours, more preferably more than 50 hours, more preferably more than 100 hours. The time needed to complete the reaction will depend on various factors such as the nature of the reagents or the reaction temperature.

Further, according to the experiments performed by the inventors, the compound of formula IV is the intermediate to the compound of formula III (see Example 2). That is, if the reaction media contains no free alcohol and the reaction is left to stand sufficient time, the compound of formula IV is formed. Under the same conditions, but having an excess of alcohol, the reaction begins forming large amounts of the corresponding compound of formula II and small amounts of the compound of formula IV. HPLC shows that the amount of the compound of formula IV remains essentially constant through time, while the amount of the compound of formula II decreases and the amount of the compound of formula III increases.

Thus it is possible to transform the isolated compound of formula IV into a compound of formula III. According to an embodiment of the invention, the compound of formula IV is further transformed into a compound of formula III, by further contacting said compound of formula IV with an alcohol.

### Transformation of compounds of formula II, III and IV

The compounds of the invention may be further manipulated. For example, the removal from the compounds of the invention of the Z group yields compounds having free hydroxyl groups on C6 and C11. For methods for the removal of the Z group, reference is made to Greene and Wuts' "Protective Groups in Organic Synthesis", John Wiley & Sons, Inc., New York, 1999. In the case of the compounds of formula III the overall result is the selective introduction of an alkoxyl group on the C4 position.

Further transformations may include protection/deprotection reactions of other positions of the compounds of the invention, e.g. protection/deprotection reactions on the different functionalities on positions 1, 2 , 3, 5, 8 and/or 9; or, where possible, hydroxyl inversion (e.g. Mitsunobu reaction).

### Compounds of formula II, III and IV

A further aspect of the present invention is a compound of formula II as defined above. According to a preferred embodiment R₁ is -OH. According to a further embodiment R₁ is -H or -OAc. As mentioned above, the process of the invention is also suitable to synthesize compounds of formula III and IV.

Thus, a further aspect of the invention is a compound of formula III as defined above with the proviso it is not 6,11-O-isopropylidene-4,9-anhydrotetrodotoxin-hydrochloride (Rajappa hydrochloride).

A further aspect of the invention is a compound of formula IV as defined above with the proviso it is not 4-O-Methyl-6,11-O-isopropylidenetetrodotoxin-hydrochloride (Gougoutas hydrochloride).

According to a preferred embodiment, R₂ and R₃ are -OH in the compounds of formula II, III and IV. According to further embodiment R₂ and R₃ are independently selected from -H or -OAc in the compounds of formula II, III and IV.

According to a preferred embodiment, R₇ and R₈ are both -H, methyl; or R₇ is -H and R₈ is selected from the group consisting of methyl, t-butyl, 2,2-dimethylpropyl, vinyl, methoxy and ethoxy; or R₇ is methyl and R₈ is selected from the group consisting of t-butyl, 4'-methoxyphenyl, ethoxy and methoxy.

### Uses of the compounds of formula II, III and IV and pharmaceutical compositions comprising the same

A further aspect of the present invention is a pharmaceutical composition comprising a compound of formula II as defined above or a pharmaceutically acceptable salt or solvate or tautomer or enantiomer thereof, and at least one pharmaceutically acceptable carrier.

A further aspect of the present invention is a pharmaceutical composition comprising a compound of formula III as defined above, or a pharmaceutically acceptable salt or solvate or tautomer or enantiomer thereof, and at least one pharmaceutically acceptable carrier.

The compounds of formula III, depending on their nature, readily sublimate under reaction conditions. Therefore, according to an embodiment of the invention, the pharmaceutical composition comprising a compound of formula III is pressurised inhalators (solution, suspension, emulsion, etc.); inhalation powders (hard capsule); pre-dispensed inhalation powders; inhalation vapours (powder, capsule, solution, tablet, ointment, liquid, etc.) or inhalation gases.

A further aspect of the present invention is a pharmaceutical composition comprising a compound of formula IV as defined above, or a pharmaceutically acceptable salt or solvate or tautomer or enantiomer thereof, and at least one pharmaceutically acceptable carrier.

The above will be referred to as the "pharmaceutical compositions of the invention".

For its administration to a subject, such as a mammal, e.g., a human, in need of treatment, the pharmaceutical compositions of the invention may be administered by any appropriate route (via), such as, oral (e.g., oral, sublingual, etc.), parenteral (e.g., subcutaneous, intramuscular, intravenous, intramuscular, etc.), rectal, nasal, topical, ophtalmic, etc.

The carriers (as defined previously in the present patent application) necessary to manufacture the desired pharmaceutical form of administration of the pharmaceutical compositions of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods and excipients to be used and processes for producing them can be found in "Tratado de Farmacia Galénica", C. Faulí i Trillo, Luzán 5, S.A. de Ediciones, 1993.

The percentage of the compound of formula I in the pharmaceutical composition will be different depending on a number of factors, such as the administration form.

In a particular embodiment, the pharmaceutical compositions of the invention comprise, as active ingredient, at least one compound of formula II, III or IV in a therapeutically effective amount. In the sense used in this description, the expression "therapeutically effective amount" refers to the quantity of active ingredient calculated to produce the desired effect, and will generally be determined, among other reasons, by the own features of the active ingredient used and the therapeutic effect to be obtained. In a particular embodiment, the dose of active ingredient administered to a subject in need of treatment for the treatment and/or prophylaxis of the conditions is within the range of 10⁻⁴ to 10³ mg/kg of body weight, preferably 10⁻¹ to 10² mg/kg of body weight.

A further aspect of the present invention is the use of a compound of formula II as described above for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction

A further aspect of the present invention is the use of a compound of formula III as described above for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

A further aspect of the present invention is the use of a compound of formula IV as described above for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

The above will be referred to as "use of the compounds of the invention".

Pain can originate for many reasons. A familiar cause is trauma, such as a sprain or muscle injury or broken bone, or from surgery. Pain due to inflammation, such as a toothache, is also familiar to many. Headache is a common experience and arises often for unknown reasons. Cancer patients may have pain for a variety of reasons. It may be due to the effects of the cancer itself, or it could result from treatment methods, such as chemotherapy or surgery. For example, after surgery a person feels pain as a result of the operation itself. Not all people with cancer have pain, and those who do are not in pain all the time.

A preferred embodiment is the use of the compounds of the invention for cancer pain or cancer treatment related pain.

A preferred embodiment is the use of the compounds of the invention for neuropathic pain.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system".

In a preferred embodiment of the use according to the invention the neuropathic pain is central pain.

According to the IASP "central pain" is defined as "a pain initiated or caused by a primary lesion or dysfunction in the central nervous system" (IASP, Classification of chronic pain, 2nd Edition, IASP Press (2002), 211).

In another embodiment of the use according to the invention the neuropathic pain is peripheral neuropathic pain or peripheral neurogenic pain.

According to the IASP "Peripheral neuropathic pain" is defined by the IASP as "pain initiated or caused by a primary lesion or dysfunction in the peripheral nervous system" and "Peripheral neurogenic pain" as "pain initiated or caused by a primary lesion, dysfunction or transitory perturbation in the peripheral nervous system" (IASP, Classification of chronic pain, 2nd Edition, IASP Press (2002), 213).

In another preferred embodiment of the use according to the invention the neuropathic pain is allodynia.

According to the IASP "allodynia" is defined as "a pain due to a stimulus which does not normally provoke pain" (IASP, Classification of chronic pain, 2nd Edition, IASP Press (2002), 210).

In another preferred embodiment of the use according to the invention the neuropathic pain is causalgia.

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

In another preferred embodiment of the use according to the invention the neuropathic pain is hyperalgesia.

According to the IASP "hyperalgesia" is defined as "an increased response to a stimulus which is normally painful (IASP, Classification of chronic pain, 2nd Edition, IASP Press (2002), 211).

In another preferred embodiment of the use according to the invention the neuropathic pain is hyperesthesia.

According to the IASP "hyperesthesia" is defined as "increased sensitivity to stimulation, excluding the senses" (IASP, Classification of chronic pain, 2nd Edition, IASP Press (2002), 211).

In another preferred embodiment of the use according to the invention the neuropathic pain is hyperpathia.

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

In another preferred embodiment of the use according to the invention the neuropathic pain is neuralgia.

According to the IASP "neuralgia" is defined as "Pain in the distribution of a nerve or nerves" (IASP, Classification of chronic pain, 2nd Edition, IASP Press (2002), 212).

In another preferred embodiment of the use according to the invention the neuropathic pain is neuritis.

According to the IASP "neuritis" is defined as "Inflammation of a nerve or nerves" (IASP, Classification of chronic pain, 2nd Edition, IASP Press (2002), 212).

In another preferred embodiment of the use according to the invention the neuropathic pain is neuropathy.

According to the IASP "neuropathy" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

Medicaments/drugs, which are frequently the subject of misuse (leading often to addiction) include opioids, especially morphine, barbiturates, cannabis, cocaine, amphetamines, phencyclidine, hallucinogens and benzodiazepines.

A preferred embodiment is the use of the compounds of the invention for the manufacture of a medicament for the treatment of drug abuse and/or drug addiction, medicament abuse and/or medicament addiction, preferably drug abuse and/or drug addiction.

A preferred embodiment is the use of the compounds of the invention for the manufacture of a medicament for the treatment of nicotine addiction.

The term "treatment" or "to treat" in the context of this specification means administration of a compound or formulation according to the invention to prevent, ameliorate or eliminate one or more symptoms associated with pain, de-addiction from the drug/medicament, or de-addiction from nicotine. Such symptoms can arise from withdrawal, or can be associated with relapse behavior, such as a craving of a subject for the drug/medicament. "Treatment" also encompasses preventing, ameliorating or eliminating the physiological sequelae of removal of the drug/medicament from an addicted subject that may cause the exhibited symptoms perceived by the subject. "Treatment" also encompasses a reduction in the amount of the drug/medicament that is consumed by a subject.

Furthermore, the terms "to treat" or "treatment" according to this invention include the treatment of symptoms of de-addiction; nicotine, drug and/or medicament dependency; nicotine, drug and/or medicament withdrawal, especially certain subtypes of de-addiction, drug dependency or nicotine withdrawal; the treatment of the consequences causing the symptoms, and the prevention or prophylaxis causing the symptoms of de-addiction, or nicotine withdrawal, as well as the prevention or the prophylaxis of the consequences causing the symptoms.

The term "effects of a de-addiction treatment" in the context of this invention is understood as including any side effect coming with any de-addiction treatment, especially any kind of withdrawal syndrome.

The term "ameliorate" in the context of this invention is understood as meaning any improvement on the situation of the patient treated - either subjectively (feeling of or on the patient) or objectively (measured parameters).

Further embodiments of the compounds of the invention are described below in the examples. It is evident for the skilled person that many variations and configurations are possible and can be obtained at will depending on the compounds selected as starting materials, the relative configurations of the functional groups present, the presence or not of chiral centers, and on the combination of reactions that are applied. The present invention encompasses all such variations (enantiomers and diastereoisomers) and possibilities.

### EXAMPLES

### General Methods and Materials

All reactions described below were carried out under argon atmosphere unless otherwise noted. The solvents used were distilled and dried under argon atmosphere before use. All starting materials were purchased commercially (Aldrich, Acros and Scharlau) and used without further purification. TLC was carried out on silica gel Merck (Kieselgel 60F-254).

¹H-RMN spectra were measured on Varian Mercury Plus 400MHz with (CH₃)₄Si as an internal reference and CD₃OD as solvent unless otherwise noted. Spectral data are reported in parts per million (δ) relative to residual signal of the solvent.

Mass spectra (DECAXP, ThermoFinnigan) were recorded in ES⁺ mode and with a direct inlet system (Ammonium Acetate, 10mM Acetonitrile).

### EXAMPLE 1: Synthesis of compounds of formula II and IV

### Preparation of 6,11 -O-isopropylidene-4,9-anhydrotetrodotoxin-hydrochloride (2) and 6,11-O-isopropylidene-4,9-anhydrotetrodotoxin-hydrochloride (3)

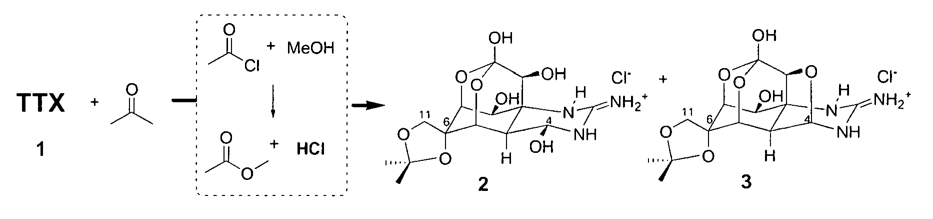

A solution of acetone (14.3 ml, 194.68 mmol), methanol (40.14 µl, 0.990 mmol) and acetyl chloride (70.32 µl, 0.988 mmol) was stirred at room temperature for 30 min. After this time, was added TTX (50 mg, 0.157 mmol). The suspension was stirred for extra 8 days. Then, it was filtered by means of a cold finger condenser to give TTX as a solid white (12 mg, 24%). To the resulting solution, diethyl ether (10 ml) was added and a white precipitate was formed; which was filtered to give a mixture of 6,1 1-*O*-isopropylidene-4,9-anhydrotetrodotoxin-hydrochloride **(2)** and 6,11-*O*-isopropylidene-4,9-anhydrotetrodotoxin-hydrochloride **(3)** (9mg, 5% **(2)** and 11% **(3)**) as white solids ([**2:3**] = 1:2). Then, the volume of the new resulting solution was reduced to 10 ml and, again, diethyl ether (10 ml) was added. The new precipitate formed was filtrated to give a mixture of 6,11-*O-*isopropylidene-4,9-anhydrotetrodotoxin-hydrochloride **(2)** and 6,11-*O*-isopropylidene-4,9-anhydrotetrodotoxin-hydrochloride **(3)** (10mg, 6% **(2)** and 12% **(3)**) as white solid **([2:3] =** 1:2). The volume of the resting solution was reduced to 10 ml and, again, diethyl ether (10 ml) was added. The new precipitate formed was filtrated to give a mixture of 6,11-*O-*isopropylidene-4,9-anhydrotetrodotoxin-hydrochloride **(2)** and 6,11-*O*-isopropylidene-4,9-anhydrotetrodotoxin-hydrochloride **(3)** (10mg, 6% **(2)** and 12% **(3)**) as a white solid ([**2:3**] = 1:2). At the end, the resting solution was concentrated under reduced pressure to give a mixture of 6,11-*O*-isopropylidene-4,9-anhydrotetrodotoxin-hydrochloride **(2)** and 6,11-*O-*isopropylidene-4,9-anhydrotetrodotoxin-hydrochloride **(3)** (10 mg, 5% **(2)** and 12% **(3)**) as a brown solid ([**2:3**] = 1:2).

### Analytic Description of the Mixture of 6,11-O-isopropylidene-4,9-anhydrotetrodotoxin hydrochloride (2) and 6,11-O-isopropylidene-4,9-anhydrotetrodotoxin-hydrochloride (3) ([2:3] = 1:2)

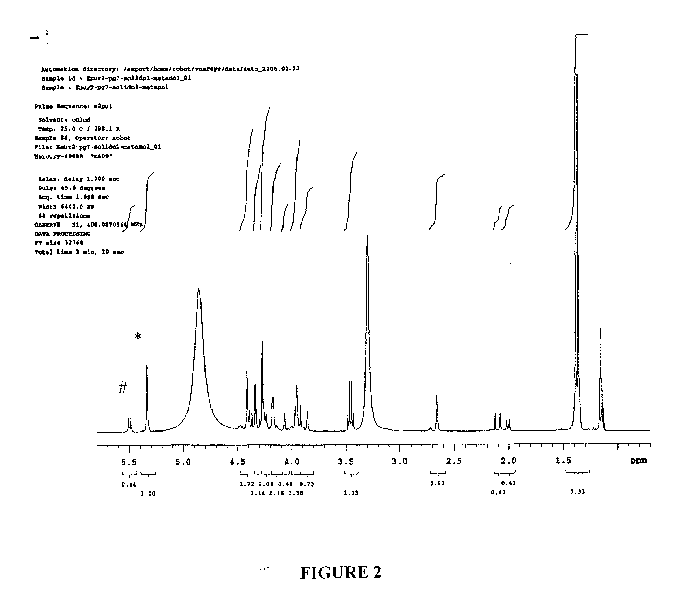

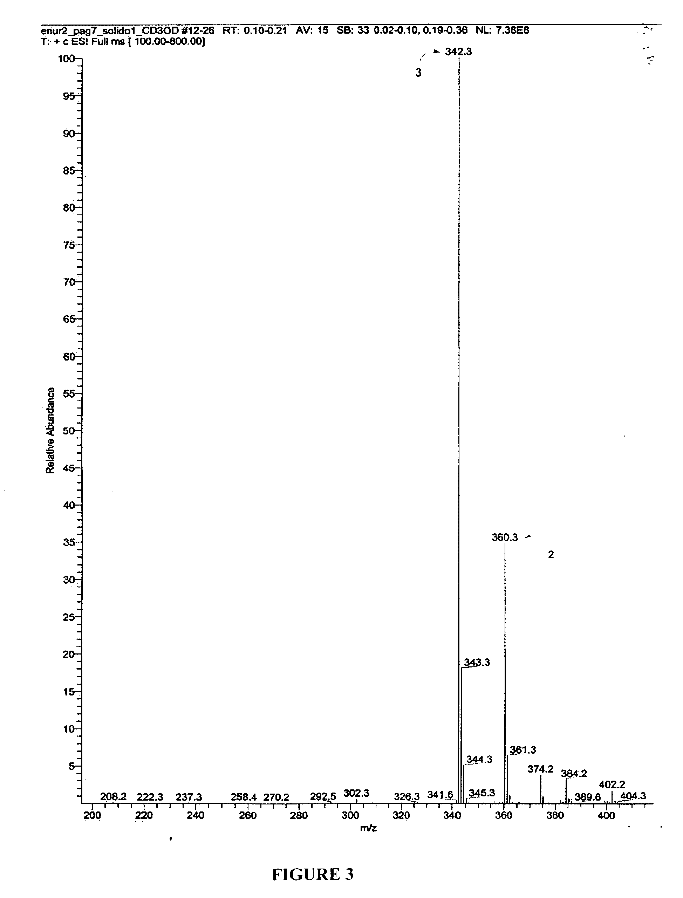

**A)** HPLC chromatogram of an aliquot (time 8 days) of the preparation reaction of compounds **2** and **3** is described in figure 1. The arrows show the peaks assigned to compounds **2** and **3** (ratio [**2:3**] = 0.46) by means of their corresponding mass spectra (see General Methods and Materials).
**B)** ¹H-RMN (Methanol-d₄) spectrum of a mixture of compounds **2** and **3** is described in figure 2 (ratio [**2:3**], aprox. 0.5; ratio calculated by the proportion of the integrals corresponding to H(4) for compounds **2** and **3**, respectively). Keys: #, signal assigned to H(4) of compound **2;** *, signal assigned to H(4) of compound **3.**
**C)** Mass spectrum of a mixture of compounds **2** and **3** is described in figure 3 (ratio [**2:3**], aprox. 0.5; determinated from ¹H-RMN and HPLC). The arrows show the peaks assigned to compounds **2** and **3**.

### Temporal set of the reaction

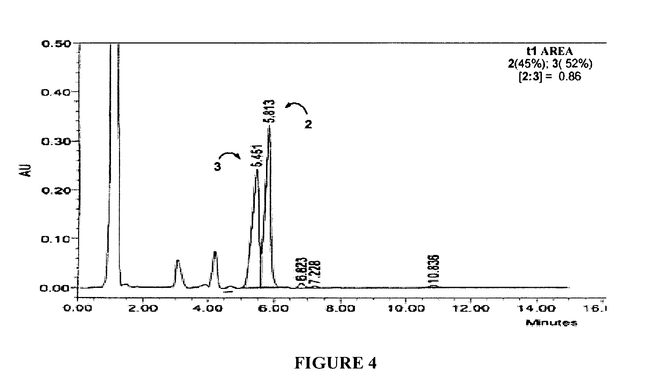

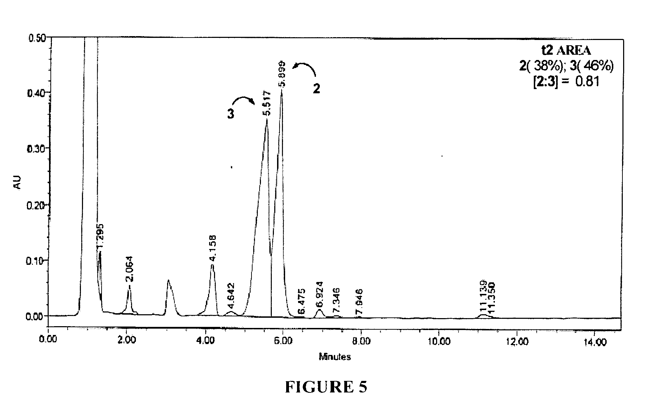

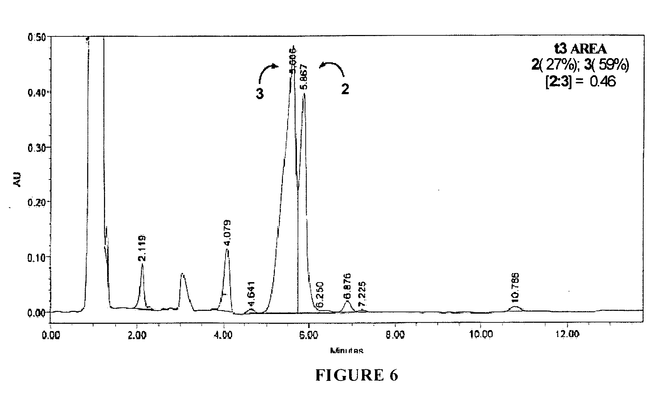

A further experiment was performed in order to determine the time sequence of the reaction. Aliquots during the reaction described above where collected and analyzed by HPLC. (The arrows show the peaks assigned to compounds **2** and **3** supported by their corresponding mass spectra). The following aliquots where collected (the HPLC in each case are shown in the figures indicated):
**a) time 26h (t1)**. See Figure 4.
**b) time 144h (t2).** See Figure 5.
**c) time 192h (t3).** See Figure 6.

### EXAMPLE 2: Synthesis of a compound of formula III 4-O-Methyl-6,11-O-isopropylidenetetrodotoxin-hydrochloride (4)

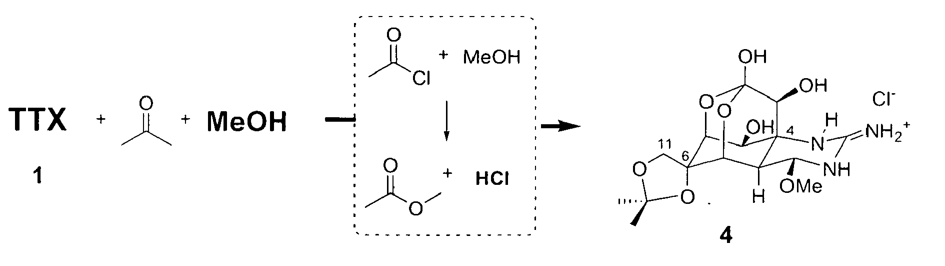

To a mixture of acetone (10 ml) and methanol (17.5 ml), acetyl chloride (423mg, 5.445 mmol) was added. The solution was stirred for 30 min. After this time, tetrodotoxin (105.20 mg, 0.330 mmol) was added at room temperature. The solution was stirred for 16h and, then, was concentrated under reduced pressure. To the resulting solution, methanol (2x 10 ml) was added and, again, concentrated under reduced pressure. Then, methanol (4ml) was added until the crude solid was completely dissolved. Diethyl ether (10 ml) was added and a white precipitate was formed. The suspension was filtrated by means of a cold finger condenser to give a 4-*O*-Methyl-6,11-*O*-isopropylidenetetrodotoxin-hydrochloride **(4)**(50 mg, 45 %) as a white solid.

### Analytic Description of 4-O-Methyl-6,11-O-isopropylidenetetrodotoxin-hydrochloride (4)

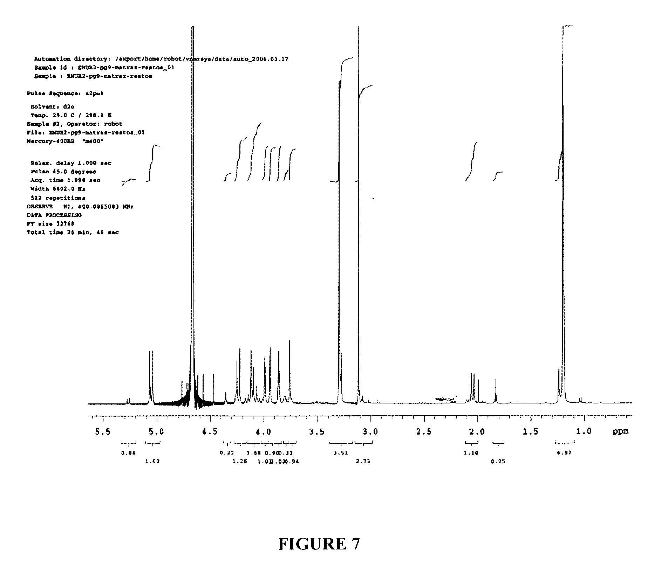

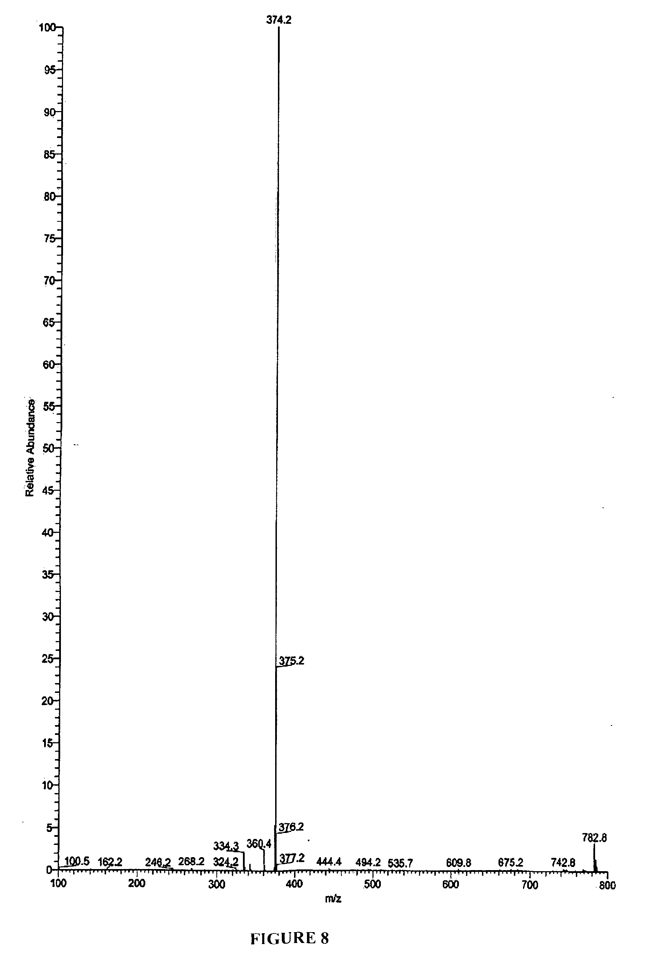

**A)** ¹H-RMN (Methanol-d₄) spectrum of compound **4** is described in figure 7.
**B)** Mass spectrum of compound **4** is described in figure 8 (see General Methods and Materials).

### Temporal set of the reaction

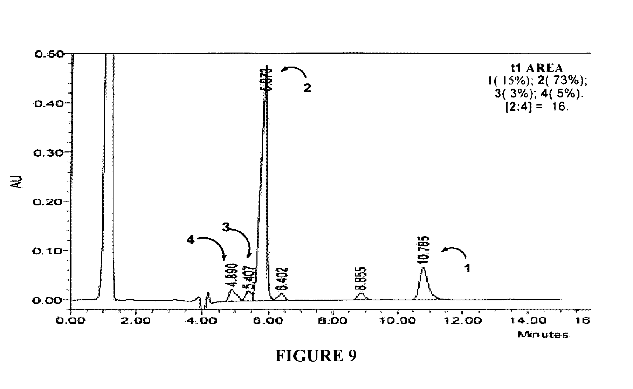

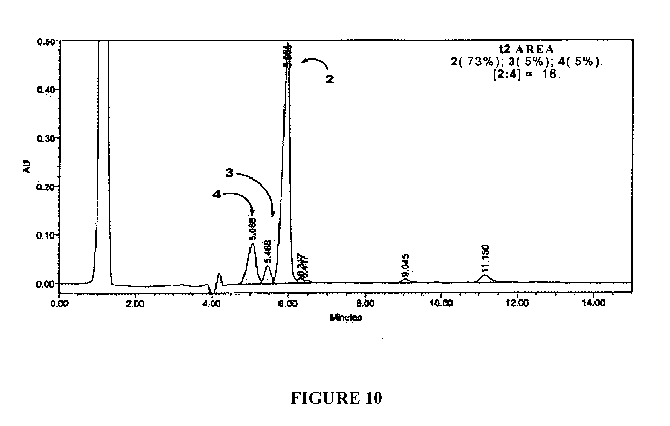

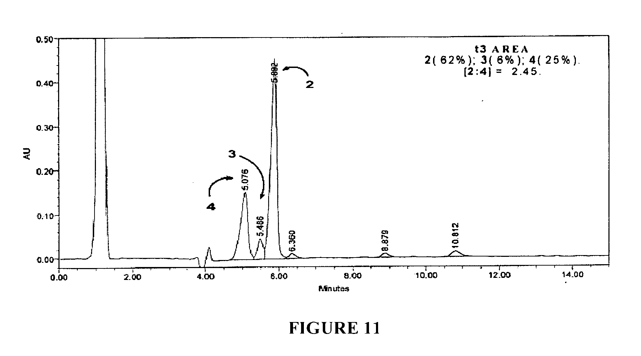

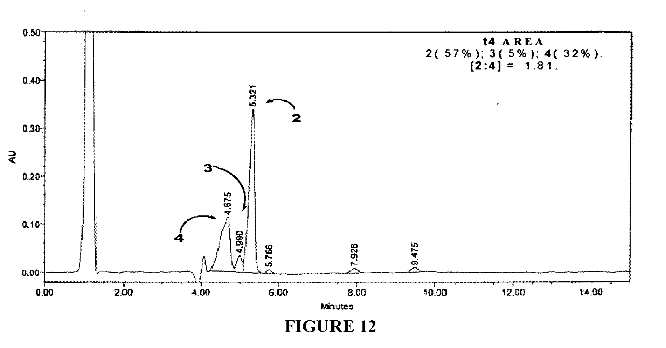

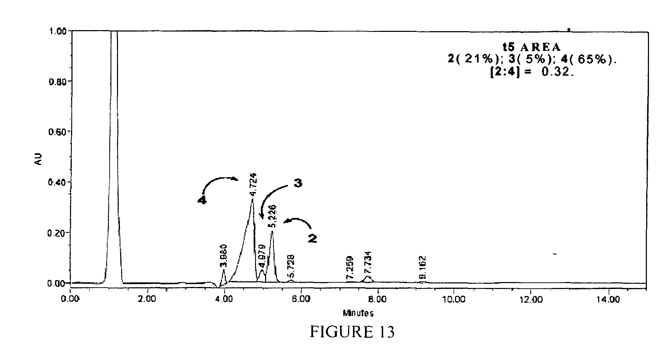

A further experiment was performed in order to determine the time sequence of the reaction. Aliquots during the reaction described above where collected and analyzed by HPLC. (The arrows show the peaks assigned to compounds **1(TTX), 2, 3** and **4** supported by their corresponding mass spectra). The following aliquots where collected (the HPLC in each case are shown in the figures indicated):
**a) time 7h (t1).** See Figure 9.
**b) time 120h (t2)**. See Figure 10.
**c) time 168h (t3)**. See Figure 11.
**d) time 216h (t4)**. See Figure 12.
**e) time 888h (t5)**. See Figure 13.

## Claims

1. A process for the synthesis of a compound of formula II: 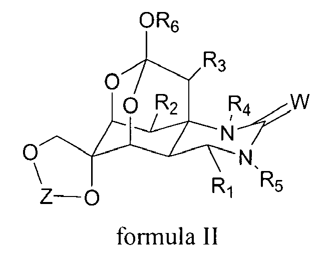 wherein
R₁ is selected from the group consisting of -H, -OH, -OC(=O)R^{a}, -OR^{a}, alkyl, -NH₂, -NHR^{b} and -NR^{b}R^{c}, wherein R^{a}, R^{b} and R^{c} are independently selected from an alkyl group;
R₂ and R₃ are independently selected from the group consisting of -H, -OH and-OC(=O)R^{a}, -OR^{b}, wherein R^{a} and R^{b} are as previously defined;
R₄ and R₅ are independently selected from the group consisting of -H and amino protecting group;
R₆ is selected from the group consisting of -H, a negative charge and a hydroxyl protecting group;
W is selected from the group consisting of NH, ⊕NH₂, O and S; and
Z is selected from the group consisting of
(R₇)(R₈)C< wherein R₇ and R₈ are each independently selected from the group consisting of alkyl, cycloalkyl, alkenyl, cycloalkenyl, alcoxyl, heterocyclyl, aryl and aralkyl; or R₇ and R₈ together form a group selected from the group consisting of cycloalkyl, heterocyclyl and cycloalkenyl; and (R₉)B<, wherein R₉ is selected from the group consisting of alkyl, cycloalkyl, alkenyl, cycloalkenyl, heterocyclyl, aryl and aralkyl;
or its salts, in particular pharmaceutically acceptable salts, or tautomers, solvates or enantiomers thereof;
comprising the step of reacting a compound of formula I 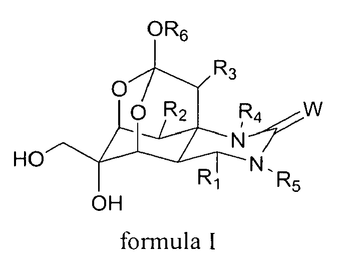 wherein
R₁, R₂, R₃, R₄, R₅, R₆ and W are as defined in formula II;
with a carbonyl or boron reagent selected from the group consisting of
- a carbonyl containing molecule of formula (R₇)(R₈)C=O or its hydrates or alkyl or aryl acetals or hemiacetals, wherein R₇ and R₈ are as previously defined;
- a boron derivative of formula R₉B(OR₁₀)₂, wherein R₁₀ are both independently selected from the group consisting of -H, alkyl, cycloalkyl, alkenyl, cycloalkenyl, heterocyclyl, aryl and aralkyl;
- a boron derivative of formula R₉B=O; and
- a boron derivative of formula 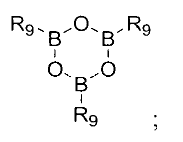 wherein R₉ is as defined above;
in the presence of a substantially anhydrous acid solution which has been generated in situ.

2. Process according to claim 1 wherein the generation of the acid comprises contacting an alcohol with an acid precursor.

3. Process according to claim 2, wherein said alcohol is an alcohol of formula R₁₁OH, wherein R₁₁ is selected from the group consisting of alkyl, cycloalkyl, alkenyl, cycloalkenyl, heterocyclyl, aryl and aralkyl.

4. Process according to any of claims 2 and 3, wherein R₁₁ is alkyl, preferably a C₁-C₆ alkyl.

5. Process according to any of claims 2 to 4, wherein said acid precursor is selected from the group consisting of
- an acyl halide of formula R₁₂COX, wherein
X is selected from the group consisting of F, Cl, Br or I; and
R₁₂ is selected from the group consisting of alkyl, cycloalkyl, alkenyl, cycloalkenyl, heterocyclyl, aryl and aralkyl;
- an acid anhydride of formula R₁₂C(=O)O(O=)CR₁₂, wherein each of R₁₂ can be the same or different as defined above; and
- an acid anhydride of formula R₁₃(O=)₂SOS(=O)₂R₁₃, wherein each of R₁₃ are independently selected from the group consisting of alkyl, preferably halogen substituted alkyl, aryl and aralkyl.

6. Process according to claim 5, wherein R₁₂ is alkyl, preferably a C₁-C₆ alkyl.

7. Process according to claim 5, wherein R₁₃ is -CF₃ or p-tolyl.

8. Process according to any of the previous claims, wherein R₁, R₂ and R₃ are -OH.

9. Process according to any of claims 1-8, wherein R₁, R₂ and R₃ are independently selected from -H or -OAc.

10. Process according to any of the previous claims, wherein the reaction time is less than 100 hours, preferably less than 50 hours, more preferably less than 40 hours, more preferably less than 30 hours, more preferably less than 25 hours.

11. Process according to any of claims 2 to 10, wherein said alcohol is in excess with regard to said acid precursor.

12. Process according to any of claims 2 to 10, wherein said alcohol and said acid precursor are in equimolar ratio.

13. Process according to any of the previous claims, wherein the compound of formula (R₇)(R₈)C=O is selected from the group consisting of formaldehyde, acetaldehyde, 2,2-dimethylpropylaldehyde, 3,3-dimethylbutirylaldehyde, 3,3-dimethyl-2-butanone, 4'-methoxyacetophenone, acrolein, acetone, ethyl acetate, methyl acetate, ethyl formate, methyl formate and 3H-Isobenzofuran-1-one, or the hydrates or alkyl or aryl acetals or hemiacetals thereof.

14. Process according to any of claims I to 12, wherein the compound of formula R₉B=O is selected from the group consisting of butylboronic acid and phenylboronic acid.

15. Process according to claim 13, wherein the compound of formula (R₇)(R₈)C=O is acetone.

16. Process for the synthesis of a compound of formula III 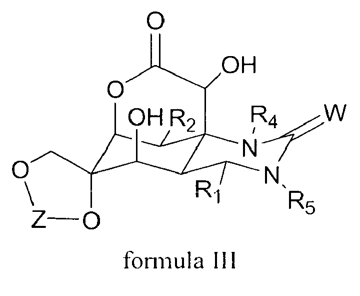 wherein
R₂, R₄, R₅ and Z are as defined in claim 1;
R₁ is alcoxyl; and
W is selected from the group consisting of -NH, O and S;
or its salts, in particular pharmaceutically acceptable salts, or tautomers, solvates or enantiomers thereof;
comprising the step of reacting a compound of formula I, wherein R₃ is -OH, with a carbonyl or boron reagent of formula (R₇)(R₈)C=O or of formula R₉B(OR₁₀)₂, or of formula R₉B=O or of formula 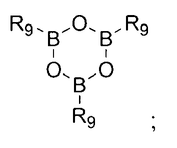 wherein the compound of formula I and the carbonyl or boron reagent are as defined in claim 1 in the presence of a substantially anhydrous acid solution which has been generated in situ and an alcohol, said alcohol being in excess with regard to the carbonyl or boron reagent.

17. Process according to claim 16, wherein said alcohol is an alcohol of formula R₁₁OH, wherein R₁₁ is selected from alkyl, cycloalkyl, alkenyl, cycloalkenyl, heterocyclyl, aryl and aralkyl.

18. Process according to claim 16, wherein R₁ is -OR₁₁, wherein R₁₁ is as defined in claim 15, preferably C₁-C₆ alkyl.

19. Process for the synthesis of a compound of formula IV 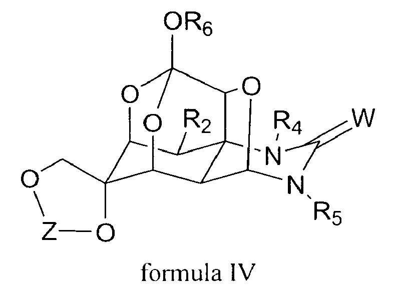 wherein
R₂, R₄, R₅, R₆ and Z are as defined in claim 1; and
W is selected from the group consisting of -NH, O and S;
or its salts, in particular pharmaceutically acceptable salts, or tautomers, solvates or enantiomers thereof;
comprising the step of reacting a compound of formula I, wherein R₃ is -OH, with a carbonyl or boron reagent of formula (R₇)(R₈)C=O or of formula R₉B(OR₁₀)₂ or of formula R₉B=O or of formula 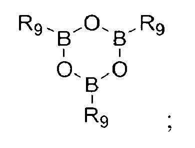 wherein the compound of formula I and the carbonyl or boron reagent are as defined in claim 1, in the presence of a substantially anhydrous acid solution which has been generated in situ, said alcohol being in equimolar ratio with regard to the carbonyl or boron reagent.

20. Process according to any of claims 16 to 19, wherein the compound is in the form of a salt, preferably a salt of and acid selected from the group consisting of XH, R₁₂C(=O)OH and R₁₃(O=)₂SOH, wherein X, R₁₂ and R₁₃ are as defined in claim 5.

21. Process according to any of claims 19 to 20 wherein the compound of formula IV is further transformed into a compound of formula III, by further contacting said compound of formula IV with an alcohol.

22. Process according to any of claims 16 to 20 wherein the reaction time is more than 5 hours, preferably more than 10 hours, more preferably more than 20 hours, more preferably more than 50 hours, more preferably more than 100 hours.

23. A compound of formula II as defined in claim 1 or its tautomers or enantiomers or solvates or salts or mixtures thereof.

24. A compound according to claim 23, wherein R₁, R₂ and R₃ are -OH.

25. A compound of formula III as defined in claim 16 with the proviso it is not 4-O-Methyl-6,11-O-isopropylidenetetrodotoxin-hydrochloride.

26. A compound of formula IV as defined in claim 19 with the proviso it is not 6,11-O-isopropylidene-4,9-anhydrotetrodotoxin-hydrochloride.

27. A compound according to any of claims 23, 25 or 26 wherein R₇ and R₈ are both -H, methyl; or R₇ is -H and R₈ is selected from the group consisting of methyl, t-butyl, 2,2-dimethylpropyl, vinyl, methoxy and ethoxy; or R₇ is methyl and R₈ is selected from the group consisting of t-butyl, 4'-methoxyphenyl, ethoxy or methoxy.

28. A pharmaceutical composition comprising a compound of formula II as defined in claim 23 or a pharmaceutically acceptable salt or solvate or tautomer or enantiomer thereof, and at least one pharmaceutically acceptable carrier.

29. A pharmaceutical composition comprising a compound of formula III as defined in claim 25, or a pharmaceutically acceptable salt or solvate or tautomer or enantiomer thereof, and at least one pharmaceutically acceptable carrier.

30. Pharmaceutical composition according to claim 29, as pressurised inhalator, inhalation powder, pre-dispensed inhalation powder, inhalation vapour or inhalation gas.

31. A pharmaceutical composition comprising a compound of formula IV as defined in claim 26, or a pharmaceutically acceptable salt or solvate or tautomer or enantiomer thereof, and at least one pharmaceutically acceptable carrier.

32. Use of a compound of formula II as defined in claim 23 for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

33. Use of a compound of formula III as defined in claim 25 for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.

34. Use of a compound of formula IV as defined in claim 26 for manufacturing a medicament for the treatment and/or prophylaxis of pain, especially cancer pain, cancer treatment related pain, moderate to severe pain, and neuropathic pain; drug abuse and/or drug addiction, medicament abuse and/or medicament addiction, preferably drug abuse and/or drug addiction; and nicotine addiction.
